# EUROPÄISCHE PATENTSCHRIFT

(11) **EP 1 126 882 B1**
(45) Veröffentlichungstag und Bekanntmachung des Hinweises auf die Patenterteilung: **30.12.2009**
(21) Anmeldenummer: 99960848.2
(22) Anmeldetag: 02.11.1999
(51) Int. Cl.: A61K 47/48, A61P 25/24

(54) **STABILE ZUBEREITUNGEN MIT HYPERFORIN**
STABLE PREPARATIONS WITH HYPERFORIN
PREPARATIONS STABLES CONTENANT DE L'HYPERFORINE

(30) Priorität: 04.11.1998 DE 19850844; 29.01.1999 DE 19903570
(43) Veröffentlichungstag der Anmeldung: 29.08.2001
(73) Patentinhaber: Dr. Willmar Schwabe GmbH & Co. KG, 76227 Karlsruhe (DE)
(72) Erfinder: STUMPF, Karl-Heinz, D-76228 Karlsruhe (DE); ERDELMEIER, Clemens, D-76139 Karlsruhe (DE); OSCHMANN, Rainer, D-76829 Landau (DE); SCHMIDT, Peter, Christian, D-72074 Tübingen (DE); ORTH, Harald, Christian, Josef, D-72070 Tübingen (DE)
(74) Vertreter: Albrecht, Thomas
(86) Internationale Anmeldenummer: PCT/DE1999/003485
(87) Internationale Veröffentlichungsnummer: WO 2000/025824

(56) Entgegenhaltungen:
- EP-A- 0 599 307
- WO-A-97/13489
- WO-A-97/22354
- WO-A-98/44936
- WO-A-99/40905
- WO-A-99/41220
- WO-A-99/64388
- DATABASE EMBASE [Online] ELSEVIER SCIENCE PUBLISHERS, AMSTERDAM, NL ORTH H.C.J. ET AL: "Stability and stabilization of hyperforin." retrieved from STN Database accession no. 2000007643 XP002136363 & DRUGS MADE IN GERMANY, (1999) 42/4 (110-113). ,
- ORTH H C J; SCHMIDT P C: "STABILITY AND STABILIZATION OF HYPERFORIN" DRUGS MADE IN GERMANY, Bd. 42, Nr. 4, 1999, Seiten 110-113, XP001056986
- J. SZEJTLI: "CYCLODEXTRINS IN PHARMACEUTICALS, pages 186-307" 1988, CYCLODEXTRIN TECHNOLOGY, KLUWER , DORDRECHT, NL , XP001194813 * Seite 186 - Seite 307 *
- UEKAMA K; HIRAYAMA F; IRIE T: "CYCLODEXTRIN DRUG CARRIER SYSTEMS" CHEMICAL REVIEWS, Bd. 98, Nr. 5, Juli 1998 (1998-07), Seiten 2045-2076, XP000771829

## Beschreibung

Durch pharmakologische und klinische Versuche ist belegt, daß Extrakte aus Johanniskraut (Hypericumextrakte) bei depressiven Verstimmungszuständen bis hin zu mittelschweren Depressionen mit Erfolg eingesetzt werden können. Die milde antidepressive Gesamtwirkung konnte jedoch noch nicht eindeutig einem oder mehreren Inhaltsstoffen zugeordnet werden; vgl. J. Hölzl, S. Sattler und H. Schütt, Johanniskraut: eine Alternative zu synthetischen Antidepressiva, Pharmazeutische Zeitung Nr. 46, 139. Jahrgang, 17. November 1994, 39593977. Allerdings gibt es Hinweise, daß zur Erzielung der Wirksamkeit Hyperforin und Adhyperforin wesentlich beiträgt (EP-A-0 599 307).

Auch die Untersuchung von reinem Hyperforin belegt, daß dieser Substanz pharmakologische Wirkungen zuzuordnen sind (S.S.Chatterjee et al.Pharmacopsychiat. 31 (1998) (Suppl.) 7-15).

Aus der Arbeit von R. Berghöfer und J. Hölzl, Deutsche Apothekerzeitung, Bd. 126, Nr. 47 (1986), Seiten 2569 - 2573, ist bekannt, daß Hyperforin in Extrakten aus gelagerter Droge bereits nach einer Woche völlig abgebaut ist, während es im Frischpflanzenextrakt stabiler sein soll. Die Verfasser vermuten, daß Frischpflanzen einen Stabilisator für Hyperforin enthalten.

J. Hölzl et al., Planta Med, Bd. 55 (1989), Seiten 601 - 602, berichten über Hypericumöl und einen vermuteten Zusammenhang zwischen der Hypericinkonzentration und der Peroxidzahl (POZ). Dem Sonnenlicht ausgesetzte Hypericumölpräparate zeigen unterschiedliche Peroxidzahlen. Nach J. Hölzl et al. besteht aber kein Zusammenhang zwischen dem POZ-Wert und der Hypericinkonzentration.

Über die Stabilität von Hypericumöl berichten P. Maisenbacher und K.-A. Kovar in Planta Med., Bd. 58 (1992), Seiten 351 - 354. Dieses Öl enthielt auch Hyperforin, das innerhalb weniger Wochen zersetzt war.

Aus der EP-A-0 599 307 (entspricht DE-OS 4 239 959) sind Hypericumextrakte und Verfahren zu ihrer Herstellung bekannt, die möglichst wenig Hypericin oder ähnliche photosensibilisierenden Verbindungen enthalten, aber dennoch die bisher dem Hypericin zugeschriebene Wirksamkeit besitzen.

Ferner ist bekannt, Hypericumöl (Johannisöl, Oleum hyperici) durch Extraktion von zerquetschten frischen Johanniskrautblüten mit einem fetten Öl wie Olivenöl, Sojaöl, Weizenkeimlingsöl oder Sonnenblumenöl herzustellen. Hypericumöl enthält variable Mengen an Hyperforin und eignet sich zur äußerlichen Behandlung von Wunden, insbesondre Brandwunden und Verschürfungen; vgl. P. Maisenbacher und K.-A. Kovar, Planta Med., Bd. 58 (1992), 351 - 354 und J. Hölzl, L. Demisch und S. Stock, Planta Med., Bd. 55 (1989), 601 - 602.

Sowohl in der Droge als auch in üblichen Hypericumextrakten nimmt der Hyperforingehalt bei normaler Lagerung innerhalb weniger Monate drastisch ab bis zum Verschwinden der Substanz; vgl. Dissertation von P. Maisenbacher, Tübingen 1991, und Dissertation von R. Berghöfer, Marburg/L. 1987. In früheren Versuchen mit öligen Hypericumextrakten konnte die Stabilität hyperforinhaltiger Zubereitungen lediglich durch Lagerung unter Argon verbessert werden; vgl. Dissertation von P. Maisenbacher, Tübingen 1991. Eine Stabilisierung durch Zusatz von Antioxidantien wie Butylhydroxytoluol (BHT) und Butylhydroxyanisol (BHA) gelang in diesen Extrakten nicht. Desgleichen bringen übliche Antioxidantien wie Oxynex LM und Oxynex 2004 keine Verbesserung der Stabilität. Bei Hypericumöl wird nach P. Maisenbacher, Dissertation, loc. cit., die beste Stabilität durch Verwendung von Octyldodecanol als. Extraktionsmittel erreicht; vgl. Dissertation P. Maisenbacher 1991, Seiten 151-154.

Aus der DE-PS 196 19 512 (WO 97/13489) und WO 98/44936 ist bekannt, daß Hyperforin durch Zusatz bestimmter Antioxidantien (organische Thiolverbindungen, Ascorbinsäure) in Extrakten stabilisierbar ist.

Reises Hyperforin weist eine ausgeprägte Instabilität auf und ist sowohl als isolierte Reinsubstanz als auch in gelöster Form nur begrenzet haltbar (C.A.J. Erdelmeier, Pharmacopsychiatry 31 (1998) (Suppl.) 2-6.) Infolge dieser Eigenschaft ist sowohl die therapeutische Verwendung von Hyperforin als auch die Verwendung als analytische Referenzsubstanz praktisch nicht möglich.

Hyperforin als Reinsubstanz kann aus Hypericumextrakten durch Anreicherung und Isolierung gewonnen werden (C.A.J. Erdelmeier, Pharmacopsychiatry 31 (1998) (Suppl.) 2-6., H.Orth, Abstractband Jahreskongreß SGPhW/DPhG, 2.-5.10.1997, Zürich P73).

Hyperforinhaltige Hypericumexträkte können mit in der Pharmazie üblichen anorganischen oder organischen Lösungsmitteln oder deren Gemische hergestellt werden (P. List und P.C. Schmidt, Technologie pflanzlicher, Arzneizubereitungen, Wissensch. Verlagsgeselischaft mbH, Stuttgart 1984).

In üblichen ethanolisch-wässrigen Hypericumextrakten und daraus hergestellten Fertigarzneimitteln ist, bezogen auf den Extrakt, in der Regel weniger als ca. 1 % Hyperforin enthalten. Nach Lagerung sinkt der Wert deutlich ab und geht je nach Lagerungsbedingungen gegen null. Man vermutet, daß Oxidationsprozesse für den Abbau des Hyperforins in der Droge und im Extrakt verantwortlich sind.

Der vorliegenden Erfindung liegt die Aufgabe zugrunde, Hyperforin
in Form seiner Extrakte und Konzentrate zu stabilisierten. Eine weitere Aufgabe der Erfindung ist es, pharmazeutische Zubereitungen bereitzustellen, die hyperforinhaltige Extrakte oder Konzentrate in stabilisierter Form enthalten. Eine weitere Aufgabe ist es, ein Verfahren zur Herstellung von stabilisiertem Hyperforin
in Form seiner Extrakte, Konzentrate und pharmazeutischen Zubereitungen bereitzustellen.

Diese Aufgaben werden durch den Gegenstand der Patentansprüche gelöst. Die Lösung beruht auf dem unerwarteten und deshalb überraschenden Befund, daß Komplexierungsmittel diese Stabilisierung bewirken. Damit ist die Möglichkeit gegeben,
Hyperforin in Extraktzubereitungen durch Komplexierung mit einem Komplexierungsmittel in stabile Zubereitungen zu überführen Dabei wird unter Komplexierung auch die Bindung an Polyhydroxyverbindungen sowie die Bildung von Einschlußverbindungen verstanden. Spezielle Beispiele für erfindungsgemäß verwendbare Komplexierungsmittel sind Kieselgele und Citronensäure.

Überraschenderweise wurde gefunden_{;} daß bei Vorliegen von Hyperforin in diesen komplexen Formen der oxidative Abbau des Hyperforins vermindert ist, ohne daß eigentliche Antioxidantien anwesend sind. Im allgemeinen wird das Komplexierungsmittel und das Hyperforin im molaren Verhältnis 1:12 bis 12:1 eingesetzt.

Stabilisierende Effekte wurden bei einzelnen komplexbildenden Hilfsstoffen, wie Kieselgel oder Citronensäure beobachtet.

Die Komplexierung des Hyperforins in Extrakten mittels der angegebenen polymeren Komplexierungsmittel kann auf einfache Weise erfolgen, z.B. durch intensives Mischen des Hyperforin-enthaltenden Extraktes mit dem polymere Komplexierungsmittel, gegebenenfalls unter Schutzgas (z.B. Stickstoff) und anschließendes Verpressen des erhaltenen Gemisches unter Zusatz weiterer Hilfsstoffe zu Tabletten. In einem weiteren Herstellungsverfahren wird der Hyperforin-enthaltende Extrakt und das polymere Komplexierungsmittel kurzzeiterhitzt und damit der Extrakt gegebenenfalls unter Schutzgas in geschmolzenes Polymer eingebettet. Nach dem Erkalten wird das erhaltene Gemisch zerkleinert und mit weiteren Hilfsstoffen abgemischt und zu Tabletten weiterverarbeitet. Schließlich besteht auch die Möglichkeit den Hyperforin-enthaltenden Extrakt und das polymere Komplexierungsmittel unter Wärmezufuhr zu mischen und ggf. unter Schutzbegasung direkt zu extrudieren. Das Extrudat kann direkt zu Granulat und dann gegebenenfalls unter Zusatz von Hilfsstoffen zu Tabletten weiterverarbeitet werden.

Die beschriebenen hyperforinhaltigen stabilisierten Konzentrate und Zubereitungen und Komplexe können anschließend in pharmazeutische Zubereitungen weiterverarbeitet werden, wobei sowohl Zubereitungen ausgehend von reinem Hyperforin als auch ausgehend von hyperforinhaltigen Extrakten oder Konzentraten oder Zubereitungen mit Hyperforinkomplexen versetzten Extrakten denkbar sind. Solche können beispielsweise wie folgt erhalten werden: Durch Nachextraktion eines alkoholischen Johanniskrautextraktes mit einem lipophilen Lösungsmittel wird ein Hyperforinkonzentrat erhalten. Dieses wird gemäß nachfolgendem Referenzbeispiel 2 mit β-1,8-CD behandelt und nach der Komplexierung dem ausextrahierten Rückstand wieder zugefügt. Diese stabilisierten Hyperforinzubereitungen können mit bekannten galenischen Hilfsstoffen wie Füll-, Binde-, Spreng- und Schmiermitteln in Tabletten, Kapseln, Dragees und bei Zusatz entsprechender anderer Hilfsstoffe in andere übliche Arzneimittelformen gebracht werden.

Die Erfindung wird anhand folgender Beispiele erläutert. Sie sind nicht einschränkend aufzufassen.

### Beispiel 1

### Stabilisierung mit Kieselgel.

Zur Herstellung der Hyperforin-Kieselgelzubereitung wird Rohhyperforin (0,1 g) in Methanol gelöst und mit Kieselgel 60 (100 g) eine Suspension gebildet. Das Methanol wird am Rotationsverdampfer abgezogen. Durch den rotierenden Rundkolben am Rotationsverdampfer kann eine Vormischung erzielt werden. Das Kieselgel 60/Rohyperforin-Gemisch wird danach im Turbula-Mischer gemischt und lyophilisiert. Die Lagerung erfolgt bei-30°C, 4°C oder 20°C.

Der Zusatz von Kieselgel 60 bewirkt in dem eingesetzten Konzentrationsbereich von 0,1 % Hyperforinanteil in der Kieselgel 60 Zubereitung innerhalb von 6 Monaten eine deutliche Stabilitätsverbesserung.

### Ergebnisse:

| | |
|---|---|
| Ausgangsgehalt: | 100 % Hyperforin in Zubereitung |
| | Nach 6 Monaten bei 4°C Lagerung 88 % vom Ausgangsgehalt |
| | Nach 6 Monaten bei 20 °C Lagerung 71 % vom Ausgangsgehalt |
| Vergleich: | Nach 6 Monaten bei 20°C Lagerung ohne Kieselgelzusatz: 34 % vom Ausgangsgehalt |

Ähnliche Ergebnisse werden mit Citronensäure als Komplexbildner erhalten.

### Referenzbeispiel 2

### Zubereitung mit ß-1,8 Cyclodextrin

Die Herstellung erfolgt, indem Rohhyperforin mit ß-1,8 Methyl-cyclodextrin im molaren Verhältnis 1:12 (Hyperforin:Cyclodextrin) in alkoholisch-wäßriger Lösung versetzt wird:
19,4 g β-1,8 Methyl-cyclodextrin (β-1,8m-CD) und 17,9 ml Hyperforinlösung (70 %) werden in 50 ml Methanol gelöst. Die Lösung wird 24 Stunden mit einem Magnetrührer bei Raumtemperatur geführt. Sodann wird die Lösung nach 24 Stunden am Rotationsverdampfer vom Methanol befreit und anschließend sofort lyophilisiert.

Ergebnisse: Diese Zubereitung ist wie die Tabelle zeigt auch bei Raumtemperatur Stabil, während nicht komplexiertes Hyperforin in dieser Zeit auf 34 % abgebaut ist.

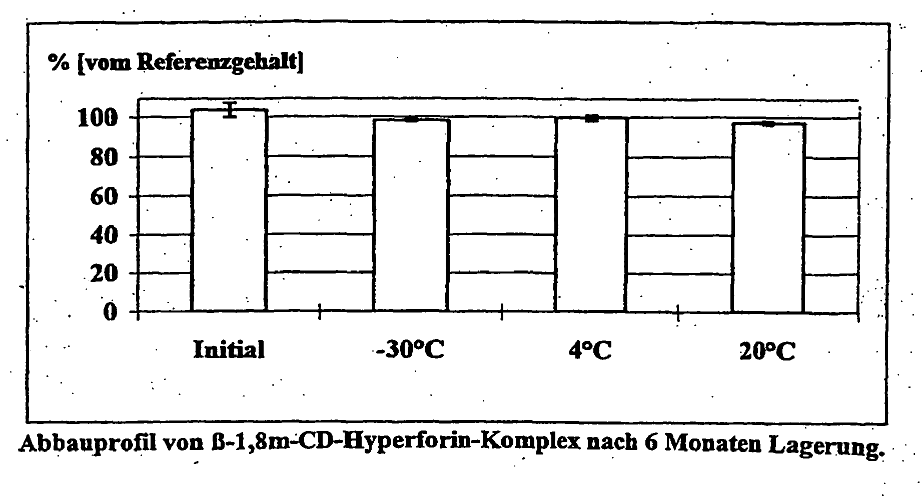

## Patentansprüche

1. Hyperforinzubereitung, enthaltend ein Komplexierungsmittel, ausgewählt aus der Gruppe bestehend aus Kieselsäuren, Citronensäure, und Gemischen davon, als Stabilisator.

2. Hyperforinzubereitung nach Anspruch 1, enthaltend als Kieselsäure Kieselgel.

3. Hyperforinzubereitung nach einem der Ansprüche 1 oder 2, wobei das Hyperforin in Form eines Extraktes oder als Konzentrat vorliegt.

4. Pharmazeutische Zubereitung, enthaltend einen hyperforinhaltigen Extrakt oder ein Konzentrat sowie ein Komplexierungsmittel nach einem der Ansprüche 1 oder 2 und gegebenenfalls weitere Stabilisierungsmittel.

5. Verfahren zur Herstellung der Zubereitung nach einem der Ansprüche 1 bis 4, umfassend das Einverleiben eines Komplexierungsmittels wie in einem der Ansprüche 1 oder 2 beschrieben in hyperforinhaltigen Extrakten oder Konzentraten oder Arzneimittel.

6. Verwendung einer Hyperforinzubereitung nach einem der Ansprüche 1 bis 3 zur Herstellung eines Arzneimittels mit antidepressiver Wirkung.

7. Verwendung von Kieselsäuren, Citronensäure, und Gemischen davon als Komplexierungsmittel in Hyperforinzubereitungen zur Stabilisierung von Hyperforin.

8. Verwendung nach Anspruch 7, wobei die Kieselsäure Kieselgel ist.

9. Verwendung nach einem der Ansprüche 7 oder 8, wobei das Hyperforin in der Hyperforinzubereitung in Form eines Extraktes oder als Konzentrat vorliegt.

## Claims

1. Hyperforin formulation comprising a complexing agent selected from the group consisting of silicic acids, citric acid and mixtures thereof as stabilizer.

2. Hyperforin formulation according to claim 1, comprising silica gel as silicic acid.

3. Hyperforin formulation according to any one of the claims 1 or 2 with the hyperforin being in form of an extract or concentrate.

4. Pharmaceutical formulation comprising an extract or concentrate comprising hyperforin as well as a complexing agent according to any one of the claims 1 or 2 and optionally additional stabilizers.

5. Process for the preparation of the formulation according to any one of the claims 1 to 4, comprising incorporating a complexing agent as described in any one of the claims 1 or 2 into extracts or concentrates or medicaments comprising hyperforin.

6. Use of a hyperforin formulation according to any one of the claims 1 to 3 for the preparation of a medicament with antidepressive effect.

7. Use of silicic acids, citric acid and mixtures thereof as complexing agents in hyperforin formulations for the stabilization of hyperforin.

8. Use according to claim 7 with the silicic acid being silica gel.

9. Use according to any one of the claims 7 or 8 with the hyperforin in the hyperforin formulation being in form of an extract or a concentrate.

## Revendications

1. Préparation d'hyperforine contenant un agent complexant, choisi parmi le groupe consistant en les acides siliciques, l'acide citrique et les mélanges de ceux-ci, en tant que stabilisant.

2. Préparation d'hyperforine selon la revendication 1, contenant le gel de silice comme l'acide silicique.

3. Préparation d'hyperforine selon l'une quelconque des revendications 1 et 2, dans laquelle l'hyperforine est présente sous forme d'extrait ou de concentré.

4. Préparation d'hyperforine contenant un extrait ou un concentré contenant de l'hyperforine et un agent complexant selon l'une quelconque des revendications 1 et 2 et éventuellement d'autres stabilisants.

5. Procédé pour la fabrication de la préparation selon l'une quelconque des revendications 1 à 4 comprenant l'étape consistant à incorporer un agent complexant, comme décrit dans une des revendications 1 et 2, dans des extraits ou concentrés ou médicaments qui contiennent de l'hyperforine.

6. Utilisation d'une préparation de l'hyperforine pour la fabrication d'un médicament avec un effet antidépressif.

7. Utilisation des acides siliciques, de l'acide citrique et des mélanges de ceux-ci en tant qu'agent complexant dans des préparations d'hyperforine pour stabiliser l'hyperforine.

8. Utilisation selon la revendication 7, dans laquelle l'acide silicique est le gel de silice.

9. Utilisation selon l'une quelconque des revendications 7 et 8, dans laquelle l'hyperforine dans la préparation d'hyperforine est sous forme d'extrait ou de concentré.
